# EUROPÄISCHE PATENTSCHRIFT

(11) **EP 1 276 455 B1**
(45) Veröffentlichungstag und Bekanntmachung des Hinweises auf die Patenterteilung: **24.12.2008**
(21) Anmeldenummer: 01945013.9
(22) Anmeldetag: 18.04.2001
(51) Int. Cl.: A61K 8/26, A61K 8/28, A61K 8/37, A61K 8/73, A61K 8/86, A61Q 15/00

(54) **WASSERFREIE ANTITRANSPIRANT-ZUSAMMENSETZUNG POLYSACCHARIDE ENTHALTEND**
ANHYDROUS ANTIPERSPIRANT COMPOSITION CONTAINING POLYSACCHARIDES
COMPOSITION ANTITRANSPIRANTE ANHYDRE CONTENANT DES POLYSACCHARIDES

(30) Priorität: 28.04.2000 DE 10021056
(43) Veröffentlichungstag der Anmeldung: 22.01.2003
(73) Patentinhaber: Henkel AG & Co. KGaA, 40589 Düsseldorf (DE)
(72) Erfinder: BANOWSKI, Bernhard, 40597 Düsseldorf (DE); HENGSTERMANN, Dieter, 46325 Borken (DE); WADLE, Armin, 40699 Erkrath (DE)
(86) Internationale Anmeldenummer: PCT/EP2001/004374
(87) Internationale Veröffentlichungsnummer: WO 2001/085117

(56) Entgegenhaltungen:
- WO-A-00/15180
- WO-A-01/47476
- WO-A-01/47488
- FR-A- 2 263 742
- GB-A- 2 084 872
- US-A- 4 743 440
- US-A- 5 176 903
- US-A- 5 292 530
- US-A- 5 626 856

## Beschreibung

Die Erfindung betrifft wasserfreie Antitranspirant-Zubereitungen zum Auftrag von Antitranspirant-Wirkstoffen auf die Haut.

Antitranspirant-Zusammensetzungen sind der Fachwelt in vielerlei Form bekannt. Als Antitranspirant-Wirkstoffe werden üblicherweise adstringierende Substanzen verwendet, beispielsweise Aluminium- und/oder Zirkoniumsalze. Die Zusammensetzungen sind als Sprays, Roll-on-Präparate, Stifte oder als Cremes im Handel. Derartige Formulierungen werden in Cosmetics: Science and Technology, "Antiperspirants and Deodorants" (Hrsg.: M. S. Balsam und E. G. Sagarin, 1972), Bd. 2, S. 373-416 von S. Plechner sowie in Cosmetics Science and Technology Series: "Antiperspirants and Deodorants" (Hrsg.: Karl Laden), 2. Auflage, S. 233 - 258 und S. 327 - 356 beschrieben.

Während der Kosmetikmarkt in den letzten Jahren vorwiegend durch Stift-Präparate beherrscht wurde, erfreuen sich cremeartige Antitranspirant-Zubereitungen zunehmender Beliebtheit. Viele cremeförmige Antitranspirant-Präparate, wie sie in EP 0 310 252, WO 91104009, WO 97/16161, WO 97/17942, WO 98151185, WO 98/27947, US 4,863,721, US 4,937,069 und US 5,292,530 beschrieben sind, werden wasserfrei formuliert. Derartige Präparate hinterlassen beim Anwender ein angenehm trockenes Hautgefühl nach dem Auftragen. Eine effektive Freisetzung der teilchenförmigen Antitranspirant-Wirkstoffe aus solchen Präparaten ist jedoch im Vergleich zu wasserhaltigen Formulierungen limitiert (vgl.: Chemistry and Technology of the Cosmetics and Toiletries Industry, Hrsg.: D. F. Williams und W. H. Schmitt, London: Blackie, 1996, 2. Auflage, S. 326) und die Präparate hinterlassen auf der Haut oft kein Frischegefühl. Wasserfreie Antitranspirant-Präparate auf Basis von flüchtigen Siliconölen haben außerdem den Nachteil, daß die dispergierten Wirkstoffe leicht zu sichtbaren Produktrückständen auf Haut und Kleidung führen. Unter Druckbelastung bei Applikation kommt es häufig zu einem Ausölen (Synärese), was die kosmetische Akzeptanz dieser Präparate beim Anwender herabsetzt. US 5626856 offenbart schweißhemmende "Soft Solids" mit einem Gehalt an einer Mischung aus Öl/Glyceridestern und teilchenförmigen Polysacchariden von 20 - 55 Gew.-%, worin das Gewichtsverhältnis von Öl/Glyceridester zu teilchenförmigen Polysacchariden von 5:1 - 1:3 beträgt, und flüchtige Siliconen in einer Menge von 15 - 50 Gew.-%.

Wasserhaltige emulsionsartige Antitranspirant-Cremes, wie sie beispielsweise in der US 4, 268, 499, WO 97/48373 und WO 98/27946 beschrieben sind, haben dagegen den Nachteil, daß sie nach dem Auftragen auf die Haut ein unangenehmes und langanhaltendes Nässegefühl hinterlassen und von den Anwendern häufig als klebrig und schmierig empfunden werden.

Es bestand die Aufgabe, eine wasserfreie Antitranspirant-Zusammensetzung mit verbesserter Antitranspirantleistung, besonders guter Hautverträglichkeit und optimierter Wirkstofffreisetzung zu entwickeln, die von der Haut schnell und möglichst rückstandsfrei absorbiert wird. Eine weitere Aufgabe war es, vorzugsweise pastöse Antitranspirant-Zusammensetzungen (Cremes) bereitzustellen, die eine gute Lagerstabilität ohne Synärese der Ölkomponenten bei Raumtemperatur sowie eine hohe Temperaturstabilität aufweisen, beim Auftragen auf die Haut keinen öligen Film hinterlassen, einen angenehm samtigen, trockenen und leichten Eindruck vermitteln und leicht abwaschbar sind.

Diese Aufgabe wurde erfindungsgemäß gelöst durch eine wasserfreie Antitranspirant-Zusammensetzung, die
a) wenigstens zwei teilchenförmige Polysaccharide und/oder Polysaccharid-Derivate in einer Gesamtmenge von 5 - 15 Gew.-%,
b) wenigstens einen adstringierenden Antitranspirant-Wirkstoff,
c) wenigstens eine Lipidkomponente mit einem Schmelzpunkt von 30 - 150°C und
d) 5 - 15 Gew.-% eines nichtionischen Emulgators mit einem HLB-Wert von 8 - 18 in einem flüssigen Trägermaterial enthält, umfassend
e) 0,1 - 35 Gew.-% flüchtige Silicone und
f) 5 - 25 Ges.-% eines hydrophilen Öls oder eines Gemisches hydrophiler Öle.

Als wasserfrei werden im Sinne der Erfindung Zusammensetzungen bezeichnet, die weniger als 5 Gew.-% Wasser (Kristallwasser nicht mit einbezogen), vorzugsweise weniger als 2 Gew.-% Wasser und insbesondere weniger als 1 Gew.-% Wasser enthalten. Ein Restanteil an Wasser kann rohstoffbedingt und daher unvermeidbar sein.

Die erfindungsgemäßen Zubereitungen sind feinteilige Dispersionen der Antitranspirant-Wirkstoffe und der Polysaccharide in einem flüssigen Trägermaterial. Prinzipiell sind verschiedene Applikationsformen möglich, beispielsweise Roll-on-Präparate, Stifte, oder pastöse/salbenartige Zubereitungen (Cremes), wobei jedoch cremeartige (pastöse) Zubereitungen erfindungsgemäß bevorzugt sind. Durch die Polysaccharid-Kombination wird ein besonders trockenes und samtiges Hautgefühl erreicht. Die erfindungsgemäße Zusammensetzung hinterläßt darüberhinaus keinen öligen Film oder weißen Rückstand auf Haut oder Kleidung, ist leicht abwaschbar, irritationsfrei und wird schnell von der Haut absorbiert.

Die erfindungsgemäßen Dispersionen sind besonders als schweißhemmende Mittel zur Applikation auf der Haut geeignet. In einer bevorzugten Ausführungsform weisen sie bei 25 °C eine Viskosität von wenigstens 200 Pa.s auf, wobei ein Bereich von 500 - 2000 Pa.s bevorzugt ist (Brookfield-RVF, Spindel TE Helipath, 4 Upm, 25° C).

Gegenstand der Erfindung ist auch ein Verfahren zur Herstellung der erfindungsgemäßen Zusammensetzungen, dadurch gekennzeichnet, daß die Wachs- und Ölkomponenten zusammen mit den Emulgatoren unter Rühren aufgeschmolzen werden, danach die pulverförmigen Bestandteile unter Rühren eingearbeitet werden, und man die Masse unter stetigem Rühren auf Raumtemperatur abkühlen läßt.

### 1. Polysaccharide

Als Polysaccharide können erfindungsgemäß natürliche und synthetische Polysaccharide und/oder deren Derivate eingesetzt werden, die teilchen- oder pulverförmig anfallen, und Homo- oder Heteroglykane der üblichen Zuckerbausteine darstellen. Sie können tierischen (z. B. Chitin, Tunicin), pflanzlichen (z. B. Stärke, Cellulose, Alginsäure), mikrobiellen, bakteriellen oder synthetischen Ursprungs sein. Hierzu gehören die teilchenförmigen Polysaccharide, die beispielsweise in "Encyclopedia of Chemical Technology", Kirk-Othmer, 3. Aufl., 1982, Bd. 3, S. 896-900 und Bd. 15, S. 439-458 beschrieben sind und diejenigen in "Polymers in Nature" (Hrsg.: E. A. MacGregor, C. T. Greenwood), John Wiley and Sons, 1980, Kapitel 6, S. 240-328 sowie in "Industrial Gums - Polysaccharides and their Derivatives" (Hrsg.: R. L. Whistier), 2. Aufl., Academic Press Inc. Erfindungsgemäß können zwei teilchenförmige Polysaccharide, ein Gemisch eines Polysaccharids und eines Polysaccharid-Derivates oder ein Gemisch zweier Pofysaccharid-Derivate verwendet werden. Synthetische Polysaccharide können aus den üblichen Zuckerbausteinen Glucose, Fructose, Mannose, Galactose, Talose, Gulose, Allose, Idose, Arabinose, Xylose, Lyxose und Ribose bzw. Gemischen davon aufgebaut sein. Ihr Molekulargewicht ist vorzugsweise größer als 5000.

Die Polysaccharide, welche die Zusammensetzungen gegen Ausölen stabilisieren und festigend wirken, werden in den erfindungsgemäßen Zubereitungen in einer Gesamtmenge von 5 - 15 Gew.-% eingesetzt. Um besonders feinteilige Dispersionen zu erhalten, kann die Verwendung von Polysacchariden mit Teilchengrößen von weniger als 100µm, insbesondere von weniger als 50 µm bevorzugt sein.

Zu den natürlich vorkommenden, teilchenförmig anfallenden Polysacchariden zählen beispielsweise Stärke und Cellulose, die Polykondensationsprodukte der D-Glucose darstellen, sowie Inulin, ein Polykondensat der D-Fructose. Die Molmasse der erfindungsgemäß verwendbaren, hochpolymeren Zucker liegt üblicherweise zwischen 5000 und mehreren Millionen. Sie verleihen den erfindungsgemäßen Zusammensetzungen einen ausgeprägt hautverträglichen Charakter und einen trockenen, samtigen Abrieb.

In einer bevorzugten Ausführungsform enthält die Antitranspirant-Zusammensetzung Polysaccharide, die ausgewählt sind aus der Gruppe der Stärke und Cellulose. Besonders bevorzugt ist die Kombination einer Stärke/eines Stärkederivates und einer Cellulose.

Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchten wie z. B. Erbsen und Bohnen, Bananen oder dem Mark bestimmter Palmensorten (z. B. Sagopalme) gewonnen werden. Erfindungsgemäß einsetzbar sind natürliche, aus Pflanzen gewonnene Stärken und/oder chemisch oder physikalisch modifizierte Stärken. Eine besonders bevorzugte Ausführungsform der Erfindung enthält eine hydrophob modifizierte Stärke. Eine Hydrophobierung läßt sich beispielsweise durch Einführung langkettiger oder verzweigter Seitenketten an einer oder mehreren der Hydroxylgruppen der Stärke erreichen. Üblicherweise handelt es sich um Ester, Ether oder Amide der Stärke mit C₁-C₄₀-Resten. Produkte, die Ester oder Ether der Stärke mit Carbonsäuren bzw. Fettalkoholen aus natürlichen Fetten und Ölen darstellen, können bevorzugt sein. Mit einem oder mehreren n-Octenylsuccinatresten veresterte Stärken sind im Sinne der Erfindung bevorzugt. Besonders vorteilhaft ist eine rieselfähige Aluminium-Octenylsuccinat-Stärke, welche z. B. von National Starch unter der Bezeichnung "Dry Flo^{®} Plus" angeboten wird. Ebenso sind modifizierte, quervernetzte Reisstärken und quervernetzte Distärkephosphorsäureester auf Basis Maisstärke einsetzbar, die von der Dr. Hauser GmbH unter der Bezeichnung Rice^{®} NS, Rice^{®} NS TC, D.S.A. 7 und unter P.F.A. 11 bzw. Mais PO4^{®} PH"B" vertrieben werden. Stärke oder Stärke-Derivate werden in den erfindungsgemäßen Zusammensetzungen in einer Menge von 0,1 - 20 Gew.-%, vorzugsweise 1 - 15 Gew.-% und insbesondere 2 - 10 Gew.-% eingesetzt.

Auch Cellulose, eine ubiquitäre pflanzliche Gerüstsubstanz, ist im Sinne der Erfindung als teilchenförmiges Polysaccharid geeignet. Sie kann in nativer Form, aber auch physikalisch oder chemisch modifiziert eingesetzt werden. Native Cellulose zeichnet sich durch eine besonders gute Hautverträglichkeit aus und ist erfindungsgemäß bevorzugt geeignet. In den erfindungsgemäßen Zusammensetzungen bewirkt sie in Kombination mit Stärke, insbesondere mit hydrophob modifizierter Stärke, besonders gute Applikationseigenschaften, ein ausgeprägt trockenes und samtiges Hautgefühl und eine Stabilisierung der Zusammensetzung gegen Ausölen (Synärese).

### 2. Lipidkomponenten mit einem Schmelzpunkt von 30 - 150° C

Als Lipidkomponenten (Definition vgl.: CD Römpp Chemie Lexikon - Version 1.0, Stuttgart/New York: Georg Thieme Verlag 1995) können erfindungsgemäß alle Fette und fettähnlichen Substanzen eingesetzt werden, die im Bereich von 30 - 150° C schmelzen. Hierzu gehören u.a. Fette (Triglyceride), Mono- und Diglyceride, Wachse, Fett- und Wachsalkohole, Fettsäuren, Ester und/oder Ether von Fettalkoholen und Fettsäuren sowie Fettsäureamide oder beliebige Gemische dieser Substanzen, solange diese im geforderten Bereich schmelzen. Diese Lipidkomponenten haben strukturgebende Eigenschaften, verleihen der Zusammensetzung die gewünschte Konsistenz und vermitteln ein besonders angenehmes Hautgefühl. Sie sind in den erfindungsgemäßen Zusammensetzungen in einer Gesamtmenge von 0,1 - 40 Gew.-%, vorzugsweise 1 - 20 Gew.-% und insbesondere 5 - 15 Gew.-% enthalten.

### Fette

Unter Fetten versteht man Triacylglycerine, also die Dreifachester von Fettsäuren mit Glycerin. Unter den Triacylglycerinen sind jene als Lipidkomponente mit einem Schmelzpunkt zwischen 30° und 150° C bevorzugt, die gesättigte, unverzweigte und unsubstituierte Fettsäurereste enthalten. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind sogenannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

In einer bevorzugten Ausführungsform ist die Lipidkomponente mit einem Schmelzpunkt von 30 - 150°C ausgewählt aus der Gruppe der Dreifachester von Glycerin mit Fettsäuren. Trigylceride wirken stark konsistenzgebend und unterstützen die Kompaktheit der Zusammensetzung. Besonders bevorzugt sind die Dreifachester von Glycerin mit C₁₈-C₆₀-Fettsäuren und insbesondere C₁₈-C₃₆-Fettsäuren. Hierzu zählt gehärtetes Rizinusöl, ein Dreifachester aus Glycerin und einer Hydroxystearinsäure, der beispielsweise unter der Bezeichnung Cutina^{®} HR im Handel ist, sowie Glycerintristearat, Glycerintribehenat (z. B. Syncrowax^{®} HRC), Glycerintripalmitat oder die unter der Bezeichnung Syncrowax^{®} HGLC bekannten Triglycerid-Gemische.

Erfindungsgemäß ebenso bevorzugt ist eine Kombination aus gehärtetem Ricinusöl und C₁₈-C₃₆-Fettsäuretriglyceriden (z. B. Syncrowax^{®} HGLC). Durch die Kombination dieser Triglyceride wird das Ausölen der Zusammensetzung weiter minimiert und die Lagerstabilität erhöht. Auch eine Kombination von gehärtetem Ricinusöl und langkettigen C₂₀-C₄₀-Fettsäuren (z. B. Performacid^{®} 350 Acid) als konsistenzgebende Lipidkomponente hat sich als vorteilhaft erwiesen.

Als Lipidkomponenten sind neben den Triglyceriden auch Mono- und Diglyceride bzw. Mischungen der Glyceride einsetzbar, solange diese Komponenten enthalten, die im Bereich von 30 - 150°C schmelzen. Zu den erfindungsgemäß bevorzugten Glyceridgemischen gehört die Kombination aus Cutina^{®} HR (gehärtetem Ricinusöl) und Novata^{®} AB (Gemisch aus C₁₂-C₁₈-Mono-, Di- und Triglyceriden).

### Fettalkohole und Fettsäuren

Zu den erfindungsgemäß einsetzbaren Fettalkoholen mit konsistenzgebenden Eigenschaften und einem Schmelzpunkt von 30 - 150°C zählen z. B. die unverzweigten C₁₄-C₅₀-Fettalkohole, insbesondere die C₁₄-C₃₀-Fettalkohole, die aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind unverzweigte, gesättigte und unsubstituierte Fettalkohole.

Aber auch verzweigte, gesättigte oder ungesättigte Fettalkohole können erfindungsgemäß verwendet werde, solange sie den geforderten Schmelzpunkt aufweisen. Erfindungsgemäß einsetzbar sind auch Fettalkoholschnitte mit einem entsprechenden Schmelzpunkt, wie sie bei der Reduktion natürlich vorkommender Fette und Öle anfallen.

Als konsistenzgebende Lipidkomponenten sind auch C₁₂-C₄₀-Fettsäuren oder deren Gemische geeignet, die im Bereich von 30 bis 150° C schmelzen. Hierzu gehören beispielsweise Laurin-, Tridecan-, Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat. Unter den Fettsäuren ist ein C₂₀-C₄₀-Fettsäuregemisch, welches unter der Bezeichnung Performacid^{®} 350 Acid im Handel ist, bevorzugt geeignet.

### Wachse

Unter Wachsen werden natürliche oder künstlich gewonnene Stoffe mit folgenden Eigenschaften verstanden: sie sind von fester bis brüchig harter Konsistenz, grob bis feinkristallin, durchscheinend bis trüb, und schmelzen oberhalb von 30° C ohne Zersetzung. Sie sind schon wenig oberhalb des Schmelzpunktes niedrigviskos und nicht fadenziehend und zeigen eine stark temperaturabhängige Konsistenz und Löslichkeit. Erfindungsgemäß verwendbar sind beispielsweise natürliche pflanzliche Wachse mit einem Schmelzpunkt von 30 - 150°C, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse.

Die Wachskomponente kann ebenso gewählt werden aus der Gruppe der Ester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren, sofern die Wachskomponente oder die Gesamtheit der Wachskomponenten im Bereich von 30 - 150° C schmelzen. Hierzu zählen beispielsweise C₁₆-C₄₀-Alkylstearate, C₂₀-C₄₀-Alkylstearate (z. B. Kesterwachs^{®} K82H), C₂₀-C₄₀-Dialkylester von Dimersäuren, C₁₈-C₃₈-Alkylhydroxystearoylstearate oder C₂₀-C₄₀-Alkylerucate. Ferner sind C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat vorteilhaft einsetzbar. Auch Siliconwachse sind gegebenenfalls vorteilhaft.

### 3. Antitranspirant-Wirkstoffe

Als Antitranspirant-Wirkstoffe eignen sich wasserlösliche adstringierende Metallsalze, insbesondere anorganische und organische Salze des Aluminiums, Zirkoniums und Zinks bzw. Mischungen dieser Salze. Unter Wasserlöslichkeit wird eine Löslichkeit von wenigstens 5 Gew.-% bei 20 °C verstanden. Erfindungsgemäß verwendbar sind beispielsweise Alaun (KAl(SO₄)₂·12 H₂O), Aluminiumsulfat, Aluminiumlactat, Natrium-Aluminium-Chlorhydroxylactat, Aluminiumchlorhydroxyallantoinat, Aluminiumchlorid, Aluminiumchlorohydrat, Aluminiumchlorohydrex-PEG, Aluminiumchlorohydrex-PG, Aluminiumsulfocarbolat, Aluminiumdichlorohydrat, Aluminiumdichlorohydrex-PEG, Aluminiumdichlorohydrex-PG, Aluminiumsesquichlorohydrate, Aluminiumsesquichlorohydrex-PEG, Aluminiumsesquichlorohydrex-PG, Aluminium-Zirkonium-Chlorohydrate (Aluminium-Zirkonium-Octachlorohydrat, Aluminium-Zirkonium-Pentachlorohydrat, Aluminium-Zirkonium-Tetrachlorohydrat, Aluminium-Zirkonium-Trichlorohydrat), Zinkchlorid, Zinksulfocarbolat, Zinksulfat, Zirkoniumchlorohydrat und Aluminium-Zirkonium-Chlorohydrat-Glycin-Komplexe, wie beispielsweise Aluminium-Zirkonium-Octachlorohydrex-Glycin, Aluminium-Zirkonium-Pentachlorohydrex-Glycin, Aluminium-Zirkonium-Tetrachlorohydrex-Glycin, Aluminium-Zirkonium-Trichlorohydrex-Glycin. Die Antitranspirant-Wirkstoffe werden in wasserfreien Cremes pulverförmig eingesetzt. Sie sind in den erfindungsgemäßen Zusammensetzungen in einer Menge von 0,1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung). In einer bevorzugten Ausführungsform enthält die Zusammensetzung eine adstringierende Aluminium- oder Aluminium-Zirkonium-Verbindung. Insbesondere geeignet sind pulverförmige Aluminiumchlorohydrate, die unter der Bezeichnung Chlorhydrol^{®}-Pulver, Reach^{®} 501 oder Micro Dry^{®} von Reheis angeboten werden, oder Aluminium-Zirkonium-Chlorohydrex-Glycin-Komplexe, die von Reheis als Rezal^{®} 36 GP Superultrafine und Rezal^{®} 33 GP vertrieben werden. Auch die Verwendung von Aluminium-Zirkonium-Pentachlorohydraten (Rezal^{®}67P) und einem Aluminium-Sesquichlorohydrat (Reach^{®} 301) kann erfindungsgemäß besonders vorteilhaft sein.

### 4. Flüssiges Trägermaterial

Als flüssiges Trägermaterial eignen sich unpolare und polare flüssige Ölkomponenten, in denen die Feststoffe möglichst homogen dispergiert sind. Erfindungsgemäß bevorzugt ist eine Kombination von unpolaren mit polaren Ölkomponenten. Zu den unpolaren flüssigen Ölkomponenten, die üblicherweise den Hauptanteil des Trägermaterials ausmachen, gehören Siliconöle und Kohlenwasserstoffe, die linear, verzweigt oder cyclisch sein können. Unter den Kohlenwasserstoffen sind beispielsweise Isohexadecan, Isododecan, Polydecene und Mineralöle wie z. B. dick- und dünnflüssige Paraffine geeignet. Um eine gute Verteilbarkeit der Zusammensetzung auf der Haut zu gewährleisten, sind dünnflüssige Trägermaterialien bevorzugt geeignet. Das Trägermaterial ist in einer Gesamtmenge von 1 - 60 Gew.-%, vorzugsweise 10 - 50 Gew.-% und insbesondere 20 - 45 Gew.-% enthalten.

### Silicone

Als unpolarer Ölkörper geeignet sind insbesondere Siliconöle. Zu diesen zählen z. B. Dialkyl- und Alkylarylsiloxane, wie beispielsweise Dimethylpolysiloxan und Methylphenylpolysiloxan, sowie deren alkoxylierte und quaternierte Analoga.

Erfindungsgemäße Zusammensetzungen sind dadurch gekennzeichnet, daß das flüssige Trägermaterial wenigstens eine flüchtige Silicon-Komponente enthält. Als flüchtig werden im Sinne der Erfindung Verbindungen bezeichnet, die sich bei Körpertemperatur verflüchtigen. Die geeigneten flüchtigen Silicone können linear, verzweigt oder cyclisch sein, sind in Todd et al., "Volatile Silicone Fluids for Cosmetics", Cosmetics and Toiletries, S. 27 - 32 (1976) beschrieben und sollen Teil der Offenbarung der vorliegenden Anmeldung sein. Bevorzugt im Sinne der Erfindung sind Silicone mit 3 - 7, insbesondere 4 - 6 Siliciumatomen. Insbesondere bevorzugt sind cyclische Polydimethylsiloxane, wie beispielsweise Octamethylcyclotetrasiloxan, Decamethylcyclopentasiloxan oder Dodecamethythyclohexasiloxan, die unter der Bezeichnung Cyclomethicone bekannt sind. Sensorisch tragen sie zu einem sehr trockenen Hautgefühl bei. Kommerziell erhältlich sind diese von G. E. Silicones als Cyclomethicone D-4 und D-5, von Dow Corning Corp. als Dow Corning^{®} 344, 345 und 244, 245, 246, von General Electric Co. als GE^{®} 7207 und 7158. Unter den linearen flüchtigen Siliconen sind diejenigen mit 1 - 7 und vorzugsweise solche mit 2 - 3 Siliciumatomen bevorzugt. Die flüchtigen Silicone sind in einer Menge von 0,1 - 35 Gew.-%, vorzugsweise 1 - 35 Gew.-% und insbesondere 10 - 35 Gew.-% enthalten.

Die Zusammensetzung kann zusätzlich nicht-flüchtige lineare Siliconöle enthalten. Entsprechende Polyalkylsiloxane, Polyalkylarylsiloxane und Polyethersiloxan-Copolymere sind in Cosmetics: Science and Technology, Hrsg.: M. Balsam und E. Sagarin, Bd. 1, 1972, S. 27-104, in US 4,202,879 und US 5,069,897 beschrieben und sollen Teil der Offenbarung der vorliegenden Anmeldung sein.

### Hydrophile Öle

Polare, flüssige Trägerkomponenten haben einen besonders großen Einfluß auf das anwendungstechnische Verhalten und die Applikationseigenschaften der Zusammensetzung. Sie begünstigen die Freisetzung des Antitranspirant-Wirkstoffes aus der hydrophoben Silicon- oder Kohlenwasserstoff-Basis. Dies ist ein wichtiges Kriterium für eine besonders gute Antitranspirantleistung. Die polaren Trägerkomponenten oder sogenannten hydrophile Öle sind organische Verbindungen, die polarer sind als Cyclomethicone. Hierzu zählen beispielsweise flüssige, verzweigte Alkanole, Alkandiole, Polyole, Etheralkohole und Dialkylether mit jeweils 12 - 40 C-Atomen oder Anlagerungsprodukte von 2 - 30 Mol Propylenoxid an ein- oder mehrwertige Alkanole mit 3 - 20 C-Atomen sowie Ester der Kohlensäure (z. B. Dioctylcarbonat). Erfindungsgemäß ebenfalls einsetzbar sind flüssige Anlagerungsprodukte von Ethylenoxid an Alkanole oder an Polyole, wie z. B. Glycerin, solange diese Produkte nicht unter die Gruppe der Emulgatoren mit einem HLB-Wert von 8 - 18 fallen (Definition: vgl. O/W-Emulgatoren).

Als flüssige verzweigte Alkanole eignen sich z. B. Guerbet-Alkohole, die eine einzige Verzweigung am β-Kohlenstoffatom aufweisen, z. B. 2-Hexyldecanol oder 2-Octyldodecanol. Weitere geeignete flüssige Alkohole sind z. B. Isotridecanol, Isohexadecanol und Gemische solcher Alkohole.

Geeignete Alkandiole sind die aus Epoxyalkanen mit 12 - 24 C-Atomen durch Ringöffnung mit Wasser erhältlichen flüssigen, vicinalen Diole. Geeignete Etheralkohole sind z. B. flüssige Mono-(C₈-C₂₂)-alkylether des Glycerins, des Ethylenglycols, des 1,2-Propylenglycols oder des 1,2-Butandiols sowie andere durch Ringöffnung aus Epoxyalkanen mit einwertigen Alkoholen erhältlichen Produkte, die 12 bis 24 C-Atome aufweisen.

Geeignete Dialkylether sind z. B. flüssige Alkylmethylether oder die Di-n-alkylether mit jeweils insgesamt 12 - 40 C-Atomen, z. B. Di-n-octylether (Cetiol^{®} OE).

Geeignete flüssige Anlagerungsprodukte von Propylenoxid an ein- oder mehrwertige Alkohole sind z.B. PPG-3-Myristyiether (Witconol^{®} APM), PPG-14-Butylether (Ucon Fluid^{®} AP), oder PPG-15-Stearylether (Arlamol^{®} E), PPG-9-Butylether (Breox^{®} B25) oder PPG-10-Butandiol (Macol^{®} 57).

Das flüssige Trägermaterial enthält zusätzlich wenigstens ein hydrophiles Öl. Vorzugsweise ist dieses ein verzweigtes Alkanol, wobei 2-Hexyldecanol oder 2-Octyldodecanol bevorzugt geeignet sind. Derartige Alkohole sind beispielsweise unter der Bezeichnung Eutanol^{®} G16 und Eutanolol^{®} G im Handel. Der Gesamtanteil des hydrophilen Öls oder des Gemisches hydrophiler Öle an der erfindungsgemäßen Zusammensetzung beträgt 5 - 25 Gew.-% und insbesondere 5 - 15 Gew.-%.

### 5. EmulgatorenlTenside

Eine weitere Hydrophilierung der Crememasse und damit auch eine optimierte Wirkstofffreisetzung und bessere Abwaschbarkeit der Rückstände von der Haut erreicht man durch Zusatz von Emulgatoren/Tensiden. Geeignet sind beispielsweise anionische und insbesondere nichtionische Tenside. Letztere zeichnen sich gegenüber anionischen Emulgatoren durch eine bessere Hautverträglichkeit aus.

### Nichtionische Emulgatoren

Zu den geeigneten nichtionischen Emulgatoren gehören beispielsweise:
(1) Anlagerungsprodukte von bis zu 70 Mol Ethylenoxid und/oder bis zu 30 Mol Propylenoxid an lineare Fettalkohole mit 8 bis 40 C-Atomen, an Fettsäuren mit 12 - 40 C-Atomen und an Alkylphenole mit 8 bis 15 C-Atomen in der Alkylgruppe.
(2) C_{12/18}-Fettsäuremono- und -diester von Anlagerungsprodukten von 1 bis 50 Mol Ethylenoxid an Glycerin.
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Acylglucamide
(6) Anlagerungsprodukte von 15 bis 60 Mol Ethylenoxid an Ricinusöl und/oder gehärtetes Ricinusöl.
(7) Polyol- und insbesondere Polyglycerinester, wie z. B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen.
(8) Anlagerungsprodukte von 2 bis 15 Mol Ethylenoxid an Ricinusöl und/oder gehärtetes Ricinusöl.
(9) Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose).
(10) Wollwachsalkohole.

(1) Polysiloxan-Polyalkyl-Polyether-Copolymere bzw. entsprechende Derivate.
(2) Mischester aus Pentaerythrit, Fettsäuren, Citronensäure und Fettalkohol gemäß DE 1165574 und/oder Mischester von Fettsäuren mit 6 bis 22 Kohlenstoffatomen, Methylglucose und Polyolen, vorzugsweise Glycerin oder Polyglycerin.
(3) Polyalkylenglykole.

### O/W-Emulgatoren

Die erfindungsgemäßen Zusammensetzungen enthalten mindestens einen nichtionischen Emulgator mit einem HLB-Wert von 8 - 18, insbesondere 13 - 18. Es kann erfindungsgemäß vorteilhaft sein, eine Kombination verschiedener Emulgatoren dieses HLB-Bereichs zu verwenden. Der Gesamtanteil der nichtionischen Emulgatoren mit einem HLB-Wert von 8 - 18 an der erfindungsgemäßen Zusammensetzung beträgt 5 - 15 Gew.-% und insbesondere 5 - 10 Gew.-%. Wie bereits erwähnt, unterstützen insbesondere O/W-Emulgatoren die schnelle und effektive Wirkstofffreisetzung. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seite 913-916, aufgelistet sind. Für ethoxylierte Produkte wird der HLB-Wert erfindungsgemäß nach folgender Formel berechnet: HLB = (100 - L): 5, wobei L der Gewichtsanteil der lipophilen Gruppen, d. h. der Fettalkyl- oder Fettacylgruppen in Gewichtsprozent, in den Ethylenoxidaddukten ist.

Zu dieser Gruppe von Emulgatoren gehören u. a. Anlagerungsprodukte von 10 - 50 Mol Ethylenoxid an Fettalkohole, Fettsäuren, Fettsäurealkanolamide, Fettsäuremonoglyceride, Sorbitanfettsäureester, Methylglucosid-Fettsäureester oder Polyglycolether-modifizierte Polysiloxane.

Vorzugsweise werden Alkoholethoxylate der Formel R¹O(CH₂CH₂O)ₙH eingesetzt, die je nach Herkunft des Alkohols z. B. als Fettalkoholethoxylate oder als Oxoalkoholethoxylate bezeichnet werden; R¹ steht für einen linearen oder verzweigten Alkyl- und/oder Alkenylrest mit 6 bis 22 Kohlenstoffatomen und n für Zahlen von 10 bis 50. Typische Vertreter sind die Addukte von durchschnittlich 10 bis 50, vorzugsweise 10 bis 30 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Auch Addukte von 10 bis 40 Mol Ethylenoxid an technische Fettalkohole mit 12 bis 18 Kohlenstoffatomen, wie beispielsweise Kokos-, Palm-, Palmkern- oder Talgfettalkohol sind geeignet. In einer besonders bevorzugten Ausführungsform enthält die erfindungsgemäße Zusammensetzung eine Emulgator-Kombination aus einem ethoxylierten Fettalkohol und einem ethoxylierten Fettsäureglycerid, beispielsweise Eumulgin^{®} B3 und Cutina^{®} E24.

### 6. Anorganische Füllstoffe

Anorganische Füllstoffe dienen nicht nur der Konsistenzgebung, sondern fungieren zum Teil auch als Geruchsabsorber. In einer bevorzugten Ausführungsform enthält die erfindungsgemäße Zusammensetzung zusätzlich wenigstens einen anorganischen Füllstoff. Zu den erfindungsgemäß einsetzbaren anorganischen Füllstoffen gehören u. a. mit der Zusammensetzung kompatible Metalloxide und -sulfate Zeolithe und Silicate. Vorzugsweise ist der anorganische Füllstoff gewählt aus der Gruppe der Silicate, die die rheologischen Eigenschaften der erfindungsgemäßen Zusammensetzung vorteilhaft unterstützen. Hierzu zählen u. a. natürliche und synthetische Siliciumdoxid-Derivate, z. B. Polykieselsäuren (z. B. Aerosil^{®}) und hydrophob modifizierte Polykieselsäuren (z. B. Aerosil^{®} R974) und Schichtsilicate, insbesondere Talkum, Montmorillonit, Kaolinit, Ilit, Beidellit, Nontronit, Saponit, Hectorit, Bentonit, Smectit und Magnesiumaluminiumsilicate. Sie werden in einer Menge von 0,1 - 25 Gew.-%, vorzugsweise 1 - 20 Gew.-% und insbesondere 5 - 15 Gew.-% eingesetzt.

Ferner können Pigmente (z. B. Titanoxid) eingesetzt werden, um die kosmetische Akzeptanz des Präparates beim Anwender zu unterstützen und organische Geruchsabsorber, wie z. B. Zinkricinoleat, Cyclodextrine und Chlorophyll.

### 7. Weitere Wirkstoffe/fakultatlve Komponenten

In die erfindungsgemäßen Zusammensetzungen können vorteilhafterweise zusätzlich die üblichen Bestandteile kosmetischer Präparate eingearbeitet werden, z. B. Stabilisatoren, Verdickungsmittel, Feuchthaltemittel, Lipidkomponenten (die nicht im Bereich von 30 - 150° C schmelzen), Hilfs- und Zusatzstoffe wie Farbstoffe und Parfümöle, Nanosphären, desodorierende Wirkstoffe, Konservierungs- und Lichtschutzmittel, Antioxidantien, Enzyme sowie Pflegestoffe und Rückfettungsmittel. Diese sind vorzugsweise in einer Menge von 0,1 - 20 Gew.-% in der Zubereitung enthalten.

### Feuchthaltemittel/Hautbefeuchtungsmittel

Die Zusammensetzung kann vorzugsweise auch ein Feuchhaltemittel oder eine Kombination aus Feuchhaltemitteln enthalten, die der Feuchtigkeitsregulierung der Haut dienen. Erfindungsgemäß geeignet sind u. a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20, PEG-32, PEG-40, PEG-55, PEG-60, PEG-75, PEG-90, PEG-100, PEG-135, PEG-150, PEG-180, PEG-200, PEG-240), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer.

Die Menge der Feuchthaltemittel in den erfindungsgemäßen Zusammensetzungen beträgt, wenn vorhanden, üblicherweise 0,1 - 15 Gew.-%, vorzugsweise 1 - 10 Gew.-% und insbesondere 2 - 8 Gew.-%, bezogen auf das Gesamtgewicht der Zusammensetzung.

### Desodorierende Wirkstoffe

Erfindungsgemäß einsetzbar sind Geruchsüberdecker (z. B. Parfüm), Geruchsabsorber oder -löscher (*vide supra*), desodorierend wirkende lonenaustauscher, keimhemmende Mittel sowie Enzyminhibitoren oder eine Kombination der genannten Wirkstoffe.

Als keimhemmende oder antimikrobielle Wirkstoffe erfindungsgemäß einsetzbar sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Cloflucarban, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Desweiteren sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Famesol, Chlorophyllin-Kupfer-Komplexe, Glycerinmonoalkylether, Carbonsäureester des Mono-, Di- und Triglycerins (z. B. Glycerinmonolaurat, Diglycerinmonocaprinat), Fettsäuren sowie Pflanzenextrakte (z. B. grüner Tee und Bestandteile des Lindenblütenöls) einsetzbar.

Weitere antibakteriell wirksame Deodorans-Wirkstoffe sind z. B. C₁₂-C₂₄-α-Hydroxyfettsäuren, α,ω-Dicarbonsäuren, Wollwachssäuren, Famesol, Lantibiotika, Imidazole, Flavonoide, Glycoglycerolipide, Sphingolipide (Ceramide), Sterine und andere Wirkstoffe, welche die Bakterienadhäsion an der Haut inhibieren, z. B. Glycosidasen, Lipasen, Proteasen, Kohlenhydrate, Di- oder Oligosaccharidfettsäureester, alkylierte Mono- oder Oligosaccharide und andere.

Zu den Enzyminhibitoren gehören Stoffe, welche die für die Schweißzersetzung verantwortlichen Enzyme, insbesondere die esterspaltenden Lipasen, hemmen, z. B. Zitronensäuretriethylester oder Zinkglycinat.

Die Menge der desodorierenden Wirkstoffe in den erfindungsgemäßen Zusammensetzungen beträgt, wenn vorhanden, 0,001 -10 Gew.-%, vorzugsweise 0,01 - 5 Gew.-% und insbesondere 0,1 - 3 Gew.-%, bezogen auf das Gesamtgewicht der Zusammensetzung.

### Konservierungsstoffe

Den erfindungsgemäßen Zusammensetzungen können fakultativ auch die üblichen Konservierungsstoffe zugesetzt werden, welche die Aufgabe haben, eine Kontamination des Produktes durch Mikroorganismen zu verhindern. Damit haben zahlreiche Konservierungsstoffe zwangsläufig auch desodorierende Eigenschaften, so daß einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z.B. Methyl-, Ethyl-, Propyl-, Isopropyl-, Butyl- Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z. B. Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylhamstoff, Imidazolidinylhamstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquatemium-42, Quatemium-8, Quaternium-14, Quaternium-15), Carbamate (z. B. lodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Triclosan, Zinkpyrithion sowie zahlreiche Peptid-Antibiotika (z. B. Nisin).

Die Menge der Konservierungsmittel in den erfindungsgemäßen Zusammensetzungen beträgt 0,001 - 10 Gew.-%, vorzugsweise 0,01 - 5 Gew.-% und insbesondere 0,1 - 3 Gew.-%, bezogen auf das Gesamtgewicht der Zusammensetzung.

### Antioxidantien

Besonders vorteilhaft werden die Antioxidantien gewählt aus der Gruppe, bestehend aus Aminosäuren (z. B. Glycin, Histidin, Tyrosin, Tryptophan) und deren Derivate, Imidazol und Imidazolderivate (z. B. Urocaninsäure), Peptide wie z. B. D,L-Camosin, D-Camosin, L-Camosin und deren Derivate (z. B. Ansecin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin, und deren Derivate, ungesättigte Fettsäuren und deren Derivate (z. B. χ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -acetat, -stearat, -dipalmitat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakaharzsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.

Die Gesamtmenge der Antioxidantien in den erfindungsgemäßen Zusammensetzungen beträgt, falls vorhanden, 0,001 - 10 Gew.-%, vorzugsweise 0,05 - 5 Gew.-% und insbesondere 0,1 - 2 Gew.-%, bezogen auf das Gesamtgewicht der Zubereitung.

Lipophile, öllösliche Antioxidantien aus dieser Gruppe sind erfindungsgemäß bevorzugt, insbesondere Tocopherol und dessen Derivate, Gallussäureester, Flavonoide und Carotinoide sowie Butylhydroxytoluollanisol. Weiterhin können öllösliche UV-Filtersubstanzen sowie alle bekannten in der Lipidschmelze löslichen kosmetischen oder dermatologischen Wirkstoffe enthalten sein. Als öllösliche Deodorantien und Konservierungsmittel sind Triethylcitrat bzw. Triclosan besonders geeignet.

Die erfindungsgemäßen Zusammensetzungen können weitere kosmetisch und dermatologisch wirksame Stoffe enthalten, wie z. B. Biotin, Pantothensäure, Sebostatika, Anti-Akne-Wirkstoffe sowie Keratolytika. Auch entzündungshemmende und die Heilung fördernde Stoffe wie z. B. Allantoin, Panthenol und verschiedene Pflanzenextrakte und Proteinhydrolysate können vorteilhaft sein.

### Erfindungsgemäße Beispiele

Die Emulgatoren, Öle und Wachskomponenten wurden zusammen auf 85°C bis zur klaren Schmelze erwärmt. In die Fettphase wurden bei 85°C unter Rühren zuerst die Polysaccharide, dann die anorganischen Bestandteile und anschließend der Antitranspirant-Wirkstoff eingearbeitet. Die heiße Masse wurde unter stetem Rühren auf Raumtemperatur abgekühlt und dann in eine zur Applikation auf der Haut geeignete Stifthülse gefüllt.

Die Mengenangaben in nachfolgenden Beispielen beziehen sich, soweit nicht anders angegeben, auf Gew.-% Aktivsubstanz der Gesamtzusammensetzung.

### Beispiel 1

- 29,4: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 8: Gew.-% modifizierte Reisstärke (z. B. D.S.A. 7)
- 2: Gew.-% Cellulose (z. B. Vitacel^{®} L 600/20 FCC)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,1: Gew.-% Butylhydroxytoluol

### Beispiel 2

- 27,4: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% gehärtetes Ricinusöl (z. B. Cutina^{®} HR)
- 3: Gew.-% C₁₈-C₂₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% modifizierte Reisstärke (z. B. D.S.A. 7)
- 2: Gew.-% Cellulose (z. B. Vitacel^{®} L 600/20 FCC)
- 5: Gew.-% Aluminium-Stärke-Octenylsuccinat (z.B. Dry Flo^{®} Plus)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,1: Gew.-% Tocopherolacetat

### Beispiel 3

- 29,3: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% gehärtetes Ricinusöl (z. B. Cutina^{®} HR)
- 3: Gew.-% C₂₀-C₄₀-Fettsäure (z. B. Performacid^{®} 350 Acid)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z.B. Dry Flo^{®} Plus)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,2: Gew.-% p-Hydroxybenzoesäurepropylester

### Beispiel 4

- 27,2: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% gehärtetes Ricinusöl (z. B. Cutina^{®} HR)
- 3: Gew.-% C₂₀-C₄₀-Fettsäure (z. B. Performacid^{®} 350 Acid)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% modifizierte Reisstärke (z. B. D.S.A. 7)
- 2: Gew.-% Cellulose (z. B. Vitacel^{®} L 600/20 FCC)
- 5: Gew.-% Aluminium-Stärke-Octenylsuccinat (z.B. Dry Flo^{®} Plus)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,2: Gew.-% Benzylalkohol
- 0,1: Gew.-% Butylhydroxytoluol

### Beispiel 5

- 29,3: Gew.-% Decamethylcyclopentasiloxan (z. B. Dow Corning^{®} 245-Fluid)
- 17: Gew.-% 2-Hexyldecanol (z. B. Eutanol^{®} G16)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-24 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z.B. Dry Flo^{®} Plus)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,2: Gew.-% Benzylalkohol

### Beispiel 6

- 28,3: Gew.-% Decamethylcyclopentasiloxan (z. B. Dow Corning^{®} 245-Fluid)
- 17: Gew.-% 2-Hexyldecanol (z. B. Eutanol^{®} G16)
- 3: Gew.-% gehärtetes Ricinusöl (z. B. Cutina^{®} HR)
- 3: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-24 Glycerylstearat (z. B. Cutina^{®} E24)
- 2: Gew.-% Cellulose (z. B. Vtacel^{®} L 600/20 FCC)
- 5: Gew.-% Aluminium-Stärke-Octenylsuccinat (z.B. Dry Flo^{®} Plus)
- 1,5: Gew.-% Silica (z.B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 0,1: Gew.-% Butylhydroxytoluol
- 0,1: Gew.-% 2,4,4'-Trichlor-2'-hydroxy-diphenylether (z.B. Irgasan^{®} DP 300)
- 1,0: Gew.-% Parfümöl

### Beispiel 7

- 39: Gew.-% Decamethylcyclopentasiloxan (z. B. Dow Corning^{®} 245-Fluid)
- 15: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 2: Gew.-% Silica (z.B. Aerosil^{®} 200)
- 5: Gew.-% Talc (z.B. Talkum Pharma^{®} G)
- 20: Gew.-% Aluminiumchlorohydrat (z.B. Micro Dry^{®} Ultrafine)

### Beispiel 8

- 32,5: Gew.-% Decamethylcyclopentasiloxan (z. B. Dow Corning^{®} 245-Fluid)
- 15: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 4: Gew.-% modifizierte Reisstärke(z. B. Rice^{®}NS)
- 5: Gew.-% Aluminium-Stärke-Octenylsuccinat (z. B. Dry Flo^{®} Plus)
- 1,5: Gew.-% Silica (z. B. Aerosil^{®}200)
- 10: Gew.-% Talc (z.B. Talkum Pharma^{®} G)
- 20: Gew.-% Aluminiumchlorohydrat (z.B. Micro Dry^{®} Ultrafine)

### Beispiel 9

- 32,5: Gew.-% Decamethylcyclopentasiloxan (z. B. Dow Corning^{®} 245-Fluid)
- 15: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 4: Gew.-% quervernetzte Maisstärke (z. B. Mais PO4^{®} PH"B")
- 5: Gew.-% Aluminium-Stärke-Octenylsuccinat (z. B. Dry Flo^{®} Plus)
- 1,5: Gew.-% Silica (z. B. Aerosil^{®}200)
- 10: Gew.-% Talc (z.B. Talkum Pharma^{®} G)
- 20: Gew.-% Aluminiumchlorohydrat (z.B. Micro Dry^{®} Ultrafine)

### Beispiel 10

- 32,5: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} R 974)
- 10: Gew.-% Talc (z.B. Talkum Pharma^{®} G)
- 20: Gew.-% Aluminiumchlorohydrat (z.B. Micro Dry^{®} Ultrafine)

### Beispiel 11

- 28,5: Gew.-% Decamethylcyclopentasiloxan (z. B. Dow Corning^{®} 245-Fluid)
- 17: Gew.-% Hexyldecanol (z. B. Eutanol^{®} G16)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)
- 1,0: Gew.-% Parfümöl

### Beispiel 12

- 29,5: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% C₁₈-C₃₆-Fettsäuretriglycerid (z. B. Syncrowax^{®} HGLC)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)

### Beispiel 13

- 27,5: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% C₁₈-C₃₆-Fettsäuretriglycerid (z. B. Syncrowax^{®} HGLC)
- 3: Gew.-% Glyceryltribehenat(z. B. Syncrowax^{®} HRC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)

### Beispiel 14 (nlcht erfindungsgemäß)

- 36,0: Gew.-% Decamethylcyclopentasiloxan (z. B. Dow Corning^{®} 245-Fluid)
- 15: Gew.-% Hexyldecanol (z. B. Eutanol^{®} G16)
- 1: Gew.-% C₁₈-C₃₆-Fettsäuretriglycerid (z. B. Syncrowax^{®} HGLC)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 0,5: Gew.-% Cellulose (z. B. Vitacel^{®} L 600/20 FCC)
- 6: Gew.-% modifizierte Reisstärke (z. B. D.S.A.7)
- 1,5: Gew.-% Silica (z. B. Aerosil^{®} 200)
- 8: Gew.-% Talc (z.B. Talkum Pharma^{®} G)
- 20: Gew.-% Aluminiumchlorohydrat (z.B. Micro Dry^{®} Ultrafine)

### Beispiel 15

- 27,5: Gew.-% Decamethylcyclopentasiloxan (z. B. Dow Corning^{®} 245-Fluid)
- 17: Gew.-% Hexyldecanol (z. B. Eutanol^{®} G16)
- 3: Gew.-% Glyceryltribehenat (z. B. Syncrowax^{®} HRC)
- 3: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Ootenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)

### Beispiel 16

- 29,5: Gew.-% Decamethylcyclopentasiloxan (z. B. Dow Corning^{®} 245-Fluid)
- 12: Gew.-% Hexyldecanol (z. B. Eutanol^{®} G16)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumsesquichlorohydrat (z.B. Reach^{®} 301)

### Beispiel 17

- 29,5: Gew.-% Decamethylcyclopentasiloxan (z. B. Dow Corning^{®} 245-Fluid)
- 5: Gew.-% Hexyldecanol (z. B. Eutanol^{®} G16)
- 10: Gew.-% Dicaprylether (z. B. Cetiol^{®} OE)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 22: Gew.-% Aluminiumchlorohydrat (z.B. Micro Dry^{®} Ultrafine)

### Beispiel 18

- 29,5: Gew.-% Decamethylcyclopentasiloxan (z. B. Dow Corning^{®} 245-Fluid)
- 10: Gew.-% Dicaprylylether (z. B. Cetiol^{®} OE)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 22: Gew.-% Aluminiumchlorohydrat (z.B. Micro Dry^{®} Ultrafine)

### Beispiel 19

- 29,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.% Dicaprylylether (z. B. Cetiol^{®} OE)
- 5: Gew.% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.% Aluminium-Stärke-Octenylsuccinat (z. B. Dry Flo^{®} Plus)
- 1,5: Gew.% Silica (z. B. Aerosil^{®} 200)
- 10: Gew.% Talc (z.B. Talkum Pharma^{®} G)
- 22: Gew.-% Aluminium-Zirkonium-Tetrachlorohydrat (z.B. Rezal^{®} 36 GP)

### Beispiel 20

- 29,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 21

- 28,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 3: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 22

- 29: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 3: Gew.-% Cellulose (z. B. Vitacel^{®} L 600/20 FCC)
- 5: Gew.-% Aluminium-Stärke-Octenylsuccinat (z. B. Dry Flo^{®} Plus)
- 10: Gew.-% Talc (z. B. Talkum Pharma^{®} G)
- 22: Gew.-% Aluminiumchlorohydrat (z.B. Micro Dry^{®} Ultrafine)
- 1: Gew.-% Parfümöl

### Beispiel 23

- 29,5: Gew.-% Decamethylcyclopentasiloxan (z. B. Dow Coming^{®} 245-Fluid)
- 5: Gew.-% Dicaprylether (z. B. Cetiol^{®} OE)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 10: Gew.-% Talc (z. B. Talkum Pharma^{®} G)
- 1,5: Gew.-% Silica (z. B. Aerosil^{®} 200)
- 22: Gew.-% Aluminiumchlorohydrat (z.B. Micro Dry^{®} Ultrafine)

### Beispiel 24

- 28,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretdglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vltacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 21: Gew.-% Aluminiumchlorohydrat (z.B. Micro Dry^{®} Ultrafine)
- 1: Gew.-% Parfümöl

### Beispiel 25

- 27,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 26

- 27,5: Gew.-% Decamethylcyclopentasiloxan (z. B. Dow Coming^{®} 245-Fluid)
- 5: Gew.-% Octyldecanol (z. B. Eutanol^{®} G)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 27

- 27,5: Gew.-% Decamethylcyclopentasiloxan (z. B. Dow Coming^{®} 245-Fluid)
- 5: Gew.-% Dicaprylylether (z. B. Cetiol^{®} OE)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 28

- 29,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} R972)
- 10: Gew.-% Talc (z. B. Talkum Pharma^{®} G)
- 22: Gew.-% Aluminiumchlorohydrat (z.B. Micro Dry^{®} Ultrafine)

### Beispiel 29

- 27,5: Gew.-% Decamethylcyclopentasiloxan (z. B. Dow Coming^{®} 245-Fluid)
- 5: Gew.-% Dicaprylylether (z. B. Cetiol^{®} OE)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 1,5: Gew.-% Silica (z. B. Aerosil^{®} R 972)
- 10: Gew.-% Talc (z. B. Talkum Pharma^{®} G)
- 22: Gew.-% Aluminiumchlorohydrat (z.B. Micro Dry^{®} Ultrafine)

### Beispiel 30

- 29,5: Gew.-% Decamethylcyclopentasiloxan (z. B. Dow Corning^{®} 245-Fluid)
- 5: Gew.-% Hexyldecanol (z. B. Eutanol^{®} G16)
- 5: Gew.-% Dicaprylylether (z. B. Cetiol^{®} OE)
- 5: Gew.-% Cocoglycerid (z. B. Novata^{®} AB)
- 6: Gew.-% gehärtetes Riciunsöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 31

- 29,5: Gew.-% Decamethylcyclopentasiloxan (z. B. Dow Coming^{®} 245-Fluid)
- 5: Gew.-% Dicaprylylether (z. B. Cetiol^{®} OE)
- 5: Gew.-% Dioctylcarbonat (z. B. Cetiol^{®} CC)
- 5: Gew.-% Cocoglycerid (z. B. Novata^{®} AB)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 32

- 29,5: Gew.-% Decamethylcyclopentasiloxan (z. B. Dow Corning^{®} 245-Fluid)
- 17: Gew.-% Octyldecanol (z. B. Eutanol^{®} G)
- 3: Gew.-% C₁₈-C₃₆-Fettsäuretriglycerid (z. B. Syncrowax^{®} HGLC)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z.B. Talkum Pharma^{®} G)
- 20: Gew.-% Aluminiumchlorohydrat (z.B. Micro Dry^{®} Ultrafine)

### Beispiel 33

- 29,5: Gew.-% Decamethylcyclopentasiloxan (z. B. Dow Coming^{®} 245-Fluid)
- 5: Gew.-% Octyldecanol (z. B. Eutanol^{®} G)
- 5: Gew.-% Dicaprylylether (z. B. Cetiol^{®} OE)
- 5: Gew.-% Polydecene (z. B. Nexbase^{®} 2004FG)
- 6: Gew.-% C₁₈-₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 1,5: Gew.-% Silica (z. B. Aerosil^{®} 200)
- 10: Gew.-% Talc (z. B. Talkum Pharma^{®} G)
- 22: Gew.-% Aluminiumchlorohydrat (z.B. Micro Dry^{®} Ultrafine)

### Beispiel 34

- 29: Gew.-% Decamethylcyclopentasiloxan (z. B. Dow Coming^{®} 245-Fluid)
- 5: Gew.-% Hexyldecanol (z. B. Eutanol^{®} G16)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 5: Gew.-% Aluminium-Stärke-Octenylsuccinat (z. B. Dry Flo^{®} Plus)
- 11: Gew.-% Talc (z. B. Talkum Pharma^{®} G)
- 22: Gew.-% Aluminiumchlorohydrat (z.B. Micro Dry^{®} Ultrafine)
- 1: Gew.-% Parfümöl

### Beispiel 35

- 28: Gew.-% Decamethylcyclopentasiloxan (z. B. Dow Corning^{®} 245-Fluid)
- 5: Gew.-% Octyldecanol (z. B. Eutanol^{®} G)
- 5: Gew.-% Cocoglycerid (z. B. Novata^{®} AB)
- 5: Gew.-% Polydecene (z. B. Nexbase^{®} 2004FG)
- 6: Gew.-% C₁₈-C₃₆-Fettsäuretriglycerid (z. B. Syncrowax^{®} HGLC)
- 3: Gew.-% Ceteareth-30 (z. B. Eumulgin^{®} B3)
- 6: Gew.-% PEG-20 Glycerylstearat (z. B. Cutina^{®} E24)
- 2: Gew.-% Cellulose (z. B. Vitacel^{®} L 600/20 FCC)
- 2: Gew.-% modifizierte Reisstärke (z. B. D.S.A.7)
- 5: Gew.-% Aluminium-Stärke-Octenylsuccinat (z. B. Dry Flo^{®} Plus)
- 11: Gew.-% Talc (z. B. Talkum Pharma^{®} G)
- 22: Gew.-% Aluminiumchtorohydrat (z.B. Micro Dry^{®} Ultrafine)

### Vergleichsbeispiel

- 32,0: Gew.-% Decamethylcyclopentasiloxan (z. B. Dow Corning^{®} 245-Fluid)
- 15: Gew.-% Hexyldecanol (z. B. Eutanol^{®} G16)
- 3: Gew.-% gehärtetes Ricinusöl (z. B. Cutina^{®} HR)
- 3: Gew.-% Ceteareth-30 (z. B. Eumulgin^{®} B3)
- 6: Gew.-% PEG-20 Glycerylstearat (z. B. Cutina^{®} E24)
- 6: Gew.-% Cellulose (z. B. Vitacel^{®} L 600/20 FCC)
- 20: Gew.-% Aluminiumchlorohydrat (z.B. Micro Dry^{®} Ultrafine)
- 15: Gew.-% Talc (z.B. Talkum Pharma^{®} G)

Alle erfindungsgemäßen Formulierungen hinterließen beim Anwender ein trockenes, samtiges Hautgefühl zogen schnell ein. Die Rezeptur des Vergleichsbeispiels (mit nur einem Polysaccharid) wurde von den Anwendern als sensorisch weniger befriedigend beurteilt und weist Stabilitätsnachteile auf.

### Anhang

1) Aerosil^{®} 200
   INCI: Silica
   Hersteller: Degussa-Hüls
2) Aerosil^{®} R 974
   INCI: Silica Dimethyl Silylate
   Hersteller: Degussa-Hüls
3) Cab-O-Sil^{®} M5
   INCI: Silica
   Hersteller: Cabot (Biesterfeld)
4) Cetiol^{®} CC
   INCI: Dioctylcarbonat
   Hersteller: Cognis Deutschland GmbH (Henkel)
5) Cetiol^{®} OE
   INCI: Dicapryl Ether
   Hersteller: Cognis Deutschland GmbH (Henkel)
6) Cetiol^{®} PGL
   INCI: Hexyldecanol, Hexydecyl Laurate
   Hersteller: Cognis Deutschland GmbH (Henkel)
7) Cutina^{®} E24
   INCI: PEG-20 Glyceryl Stearate
   Hersteller: Cognis Deutschland GmbH (Henkel)
8) Cutina^{®} HR
   INCI: Hydrogenated Castor Oil
   Hersteller: Cognis Deutschland GmbH (Henkel)
9) Dow Corning^{®} DC245
   INCI: Cyclomethicone
   Hersteller: Dow Coming
10) D.S.A. 7
   Modifizierte Reisstärke, nicht quervernetzt
   Hersteller: Dr. Hauser GmbH
11) Dry Flo^{®} Plus
   INCI: Aluminium Octenyl Succinate
   Hersteller: National starch
12) Eumulgin^{®} B3
   INCI: Ceteareth-30
   Hersteller: Cognis Deutschland GmbH (Henkel)
13) Eutanol^{®} G16
   INCI: Hexyldecanol
   Hersteller: Cognis Deutschland GmbH (Henkel)
14) Eutanol^{®} G
   INCI: Octyldodecanol
   Hersteller: Cognis Deutschland GmbH (Henkel)
15) Lorol^{®} C18
   INCI: Stearyl Alcohol
   Hersteller: Cognis Deutschland GmbH (Henkel)
16) Mais PO4^{®} PH"B"
   INCI: Distarch Phosphate
   Hersteller: Dr. Hauser GmbH
17) Nexbase^{®} 2004 FG
   INCI: Polydecene
   Hersteller: Fortum (Nynäs GmbH)
18) Novata^{®} AB
   INCI: Cocoglycerides
   Hersteller: Cognis Deutschland GmbH (Henkel)
19) Micro Dry^{®} UF
   INCI: Aluminium Chlorohydrate
   Hersteller: Reheis
20) Performacid^{®} 350 Acid
   INCI: C₂₀-C₄₀ Acid (and) Polyethylene
   Hersteller: New Phase Technologies
21) Reach^{®} 301
   INCI: Aluminiumsesquichlorohydrate
   Hersteller: Reheis
22) Rezal^{®} 36GP
   INCI: Aluminium-Zirconium Tetrachlorohydrex Glycine
   Hersteller: Reheis
23) Rice^{®} NS
   Quervernetzte Stärke
   Hersteller: Dr. Hauser GmbH
24) Syncrowax^{®} HGLC
   INCI: C₁₈-C₃₆ acid triglyceride
   Hersteller: Croda
25) Syncrowax^{®} HRC
   INCI: Glyceryl Tribehenate
   Hersteller: Croda
26) Talkum Pharma^{®} G
   INCI: Talc
   Hersteller: China National Metals & Minerals (Erbslöh)
27) Ucon^{®} AP
   INCI: PPG-14 Butyl ether
   Hersteller: Amerchol (Union Carbide)
28) Vitacel^{®} L 600/20 FCC
   Cellulosepulver
   Hersteller: J. Rettenmaier & Söhne

## Patentansprüche

1. Wasserfreie Antitranspirant-Zusammensetzung, die
a) wenigstens zwei teilchenförmige Polysaccharide und/oder Polysaccharid-Derivate in einer Gesamtmenge von 5 - 15 Gew.-%,
b) wenigstens einen adstringierenden Antitranspirant-Wirkstoff,
c) wenigstens eine Lipidkomponente mit einem Schmelzpunkt von 30 - 150°C und
d) 5 - 15 Gew.-% eines nichtionischen Emulgators mit einem HLB-Wert von 8 - 18
in einem flüssigen Trägermaterial enthält, umfassend
e) 0,1 - 35 Gew.-% flüchtige Silicone und
f) 5 - 25 Gew.-% eines hydrophilen Öls oder eines Gemisches hydrophiler Öle.

2. Zusammensetzung gemäß Anspruch 1, **dadurch gekennzeichnet, dass** die Polysaccharide ausgewählt sind aus der Gruppe der Stärke und Cellulose und/oder deren Derivate.

3. Zusammensetzung gemäß Anspruch 1 oder 2, **dadurch gekennzeichnet, dass** die Stärke hydrophob modifiziert ist.

4. Zusammensetzung gemäß einem der Ansprüche 1 bis 3, **dadurch gekennzeichnet, dass** der Antitranspirant-Wirkstoff eine adstringierende Aluminium- und/oder Aluminium-Zirkonium-Verbindung ist.

5. Zusammensetzung gemäß einem der Ansprüche 1 bis 4, **dadurch gekennzeichnet, dass** die Lipidkomponente mit einem Schmelzpunkt von 30 - 150°C ausgewählt ist aus der Gruppe der Dreifachester von Glycerin mit Fettsäuren, Mono- und Diglyceriden, Wachsen, Fett- und Wachsalkoholen, Fettsäuren, Estern und/oder Ethern von Fettalkoholen und Fettsäuren sowie Fettsäureamiden oder beliebigen Gemischen dieser Substanzen, solange diese im geforderten Bereich von 30 - 150°C schmelzen.

6. Zusammensetzung gemäß Anspruch 5, **dadurch gekennzeichnet, dass** das Fettsäuretriglycerid mit einem Schmelzpunkt von 30 - 150°C ausgewählt ist aus den Dreifachestern von Glycerin mit C₁₈-C₆₀-Fettsäuren und insbesondere C₁₈C₃₆-Fettsäuren, gehärtetem Rizinusöl, Glycerintristearat, Glycerintribehenat und Glycerintripalmitat.

7. Zusammensetzung gemäß Anspruch 5, **dadurch gekennzeichnet, dass** die Lipidkomponente mit einem Schmelzpunkt von 30 - 150°C ausgewählt ist aus unverzweigten C₁₄-C₅₀-Fettalkoholen, insbesondere den C₁₄-C₃₀-Fettalkoholen, besonders bevorzugt den unverzweigten, gesättigten und unsubstituierten Fettalkoholen, und C₁₂-C₄₀-Fettsäuren oder deren Gemischen, beispielsweise Laurin-, Tridecan-, Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierten Fettsäuren, 12-Hydroxystearinsäure, und den Amiden oder Monoethanolamiden der Fettsäuren, sowie einem C₂₀-C₄₀-Fettsäuregemisch, weiterhin ausgewählt aus natürlichen pflanzlichen Wachsen, tierischen Wachsen, Mineralwachsen, petrochemischen Wachsen, chemisch modifizierten Wachsen und synthetischen Wachsen, Estern aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren, insbesondere C₁₆-C₄₀-Alkylstearaten, C₂₀-C₄₀-Alkylstearaten, C₂₀-C₄₀-Dialkylestern von Dimersäuren, C₁₆-C₃₆-Alkylhydroxystearoylstearaten oder C₂₀-C₄₀-Alkylerucaten, C₃₀-C₅₀-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglycoldi(12-hydroxystearat), Stearylstearat, Palmitylstearat, Stearylbehenat, Cetylester, Cetearylbehenat, Behenylbehenat, sowie aus Siliconwachsen.

8. Zusammensetzung gemäß einem der Ansprüche 1 bis 7, **dadurch gekennzeichnet, dass** die Lipidkomponente mit einem Schmelzpunkt von 30 - 150°C in einer Gesamtmenge von 0,1 - 40 Gew.-%, vorzugsweise 1 - 20 Gew.-% und insbesondere 5 - 15 Gew.-%, enthalten ist.

9. Zusammensetzung gemäß einem der Ansprüche 1 bis 8, **dadurch gekennzeichnet, dass** das flüssige Trägermaterial in einer Gesamtmenge von 10 - 60 Gew.-%, vorzugsweise 10 - 50 Gew.-% und insbesondere 20 - 45 Gew.-%, enthalten ist.

10. Zusammensetzung gemäß einem der Ansprüche 1 bis 9, **dadurch gekennzeichnet, dass** das hydrophile Öl ausgewählt ist aus flüssigen verzweigten Alkanolen, Alkandiolen, Polyolen, Etheralkoholen und Dialkylethern mit jeweils 12 - 40 C-Atomen, Anlagerungsprodukten von 2 - 30 Mol Propylenoxid an ein- oder mehrwertige Alkanole mit 3 - 20 C-Atomen sowie Estern der Kohlensäure, z. B. Dioctylcarbonat, weiterhin aus flüssigen Anlagerungsprodukten von Ethylenoxid an Alkanole oder an Polyole, wie z. B. Glycerin, solange diese Produkte nicht unter die Gruppe der Emulgatoren mit einem HLB-Wert von 8 - 18 fallen, weiterhin aus Guerbet-Alkohole, die eine einzige Verzweigung am β-Kohlenstoffatom aufweisen, z. B. 2-Hexyldecanol oder 2-Octyldodecanol, weiterhin aus Isotridecanol, Isohexadecanol und Gemischen solcher Alkohole.

11. Zusammensetzung gemäß einem der Ansprüche 1 bis 10, **dadurch gekennzeichnet, dass** der nichtionische Emulgator mit einem HLB-Wert von 8 - 18 ausgewählt ist aus den Anlagerungsprodukten von 10 - 50 Mol Ethylenoxid an Fettalkohole, Fettsäuren, Fettsäurealkanolamide, Fettsäuremonoglyceride, Sorbitanfettsäureester, Methylglucosid-Fettsäureester oder Polyglycolether-modifizierte Polysiloxane.

12. Zusammensetzung gemäß einem der Ansprüche 1 bis 11, **dadurch gekennzeichnet, dass** zusätzlich wenigstens ein anorganischer Füllstoff enthalten ist.

13. Zusammensetzung gemäß einem der Ansprüche 1 bis 12, **dadurch gekennzeichnet, dass** der anorganische Füllstoff aus der Gruppe der Silikate gewählt ist.

14. Zusammensetzung gemäß einem der Ansprüche 1 bis 13, **dadurch gekennzeichnet, dass** sie bei 25°C eine Viskosität von wenigstens 200 Pa.s aufweist, gemessen mit Brookfield-RVF, Spindel TE, Helipath, 4 Upm.

15. Verwendung einer Zusammensetzung gemäß einem der Ansprüche 1 bis 14 als schweißhemmendes Mittel zur Applikation auf der Haut.

16. Verfahren zur Herstellung einer Zusammensetzung gemäß einem der Ansprüche 1 bis 14, **dadurch gekennzeichnet, dass** die Wachs- und Ölkomponenten zusammen mit den Emulgatoren unter Rühren aufgeschmolzen werden, danach die pulverförmigen Bestandteile unter Rühren eingearbeitet werden, und man die Masse unter stetigem Rühren auf Raumtemperatur abkühlen lässt.

## Claims

1. Water-free antiperspirant composition, **characterized in that** it contains:
a) at least two particulate polysaccharides and/or polysaccharide derivatives in a total quantity of 5 to 15% by weight,
b) at least one astringent antiperspirant agent,
c) at least one lipid component with a melting point of 30 to 150°C, and
d) 5 to 15% by weight of a nonionic emulsifier with an HLB value of 8 to 18,
in a liquid carrier material, comprising
e) 0.1 to 35% by weight of volatile silicones and
f) 5 to 25% by weight of a hydrophilic oil or a mixture of hydrophilic oils.

2. A composition as claimed in claim 1, **characterized in that** the polysaccharides are selected from the group consisting of starch and cellulose and/or derivatives thereof.

3. A composition as claimed in claim 1 or 2, **characterized in that** the starch is hydrophobically modified.

4. A composition as claimed in any of claims 1 to 3, **characterized in that** the antiperspirant agent is an astringent aluminium and/or aluminium zirconium compound.

5. A composition as claimed in any of claims 1 to 4, **characterized in that** the lipid component with a melting point of 30 to 150°C is selected from the group consisting of the triple esters of glycerol with fatty acids, mono- and diglycerides, waxes, fatty and wax alcohols, fatty acids, esters and/or ethers of fatty alcohols and fatty acids and also fatty acid amides or any mixtures of these substances providing they melt in the stipulated range of 30 to 150°C.

6. A composition as claimed in claim 5, **characterized in that** the fatty acid triglyceride with a melting point of 30 to 150°C is selected from the group consisting of triple esters of glycerol with C₁₈₋₆₀ fatty acids, more especially with C₁₈₋₃₆ fatty acids, hydrogenated castor oil, glycerol tristearate, glycerol tribehenate and glycerol tripalmitate.

7. A composition as claimed in claim 5, **characterized in that** the lipid component with a melting point of 30 to 150°C is selected from the group consisting of unbranched C₁₄₋₅₀ fatty alcohols, more particularly the C₁₄₋₃₀ fatty alcohols, especially preferred the unbranched, saturated and unsubstituted fatty alcohols, and C₁₂₋₄₀ fatty acids or mixtures thereof, for example, lauric, tridecanoic, myristic, pentadecanoic, palmitic, margaric, stearic, nonadecanoic, arachic, behenic, lignoceric, cerotic, melissic, erucic and elaeostearic acid and substituted fatty acids, 12-hydroxystearic acid, and the amides or monoethanolamides of the fatty acids, a C₂₀₋₄₀ fatty acid mixture, furthermore selected from natural vegetable waxes, animal waxes, mineral waxes, petrochemical waxes, chemically modified waxes and synthetic waxes, esters of saturated and/or unsaturated, branched and/or unbranched alkanecarboxylic acids and saturated and/or unsaturated, branched and/or unbranched alcohols, from the group of esters of aromatic carboxylic acids, dicarboxylic acids, tricarboxylic acids and hydroxycarboxylic acids (for example 12-hydroxystearic acid) and saturated and/or unsaturated, branched and/or unbranched alcohols and also from the group of lactides of long-chain hydroxycarboxylic acids, especially C₁₆₋₄₀ alkyl stearates, C₂₀₋₄₀ alkyl stearates, C₂₀₋₄₀ dialkyl esters of dimer acids, C₁₈₋₃₈ alkyl hydroxystearoyl stearates or C₂₀₋₄₀ alkyl erucates, C₃₀₋₅₀ alkyl beeswax, tristearyl citrate, triisostearyl citrate, stearyl heptanoate, stearyl octanoate, trilauryl citrate, ethylene glycol dipalmitate, ethylene glycol distearate, ethylene glycol di(12-hydroxystearate), stearyl stearate, palmityl stearate, stearyl behenate, cetyl ester, cetearyl behenate behenyl behenate, and from silicone waxes.

8. A composition as claimed in any of claims 1 to 7, **characterized in that** the lipid component with a melting point of 30 to 150°C is present in a total quantity of 0.1 to 40% by weight, preferably 1 to 20% by weight and more particularly 5 to 15% by weight.

9. A composition as claimed in any of claims 1 to 8, **characterized in that** the liquid carrier material is present in a total quantity of 10 to 60% by weight, preferably 10 to 50% by weight and more particularly 20 to 45% by weight.

10. A composition as claimed in any of claims 1 to 9, **characterized in that** the hydrophilic oil is selected from liquid branched alkanols, alkanediols, polyols, ether alcohols and dialkyl ethers, each containing 12 to 40 carbon atoms, products of the addition of 2 to 30 moles of propylene oxide onto mono- or polyvalent alkanols containing 3 to 20 carbon atoms, furthermore esters of carbonic acid, for example dioctyl carbonate, furthermore from liquid addition products of ethylene oxide onto alkanols or polyols such as, for example, glycerol, providing these products do not belong to the group of emulsifiers with an HLB value of 8 to 18, furthermore from Guerbet alcohols with a single branch at the β-carbon atom, for example 2-hexyl decanol or 2-octyl dodecanol, furthermore from isotridecanol, isohexadecanol and mixtures of those alcohols.

11. A composition as claimed in any of claims 1 to 10, **characterized in that** the non-ionic emulsifier with an HLB value of 8 to 18 is selected from products of the addition of 10 to 50 moles of ethylene oxide onto fatty alcohols, fatty acids, fatty acid alkanolamides, fatty acid monoglycerides, sorbitan fatty acid esters, methyl glucoside fatty acid esters or polyglycolether-modified polysiloxanes.

12. A composition as claimed in any of claims 1 to 11, **characterized in that** at least one inorganic filler is additionally present.

13. A composition as claimed in any of claims 1 to 12, **characterized in that** the inorganic filler is selected from the group of silicates.

14. A composition as claimed in any of claims 1 to 13, **characterized in that** it has a viscosity at 25°C of at least 200 Pa.s (measured with Brookfield RVF, spindle TE, Helipath, 4 r.p.m.).

15. The use of the composition claimed in any of claims 1 to 14 as a perspiration inhibitor for application to the skin.

16. A process for the production of the composition claimed in any of claims 1 to 14, **characterized in that** the wax and oil components are melted with stirring together with the emulsifiers, after which the powder-form constituents are incorporated with stirring and the whole is left to cool to room temperature with continuous stirring.

## Revendications

1. Composition anti-transpirante anhydre qui contient
a) au moins deux polysaccharides et/ou dérivés de polysaccharides sous forme de particules en une quantité totale de 5 à 15% en poids,
b) au moins une substance active anti-transpirante astringente,
c) au moins un composant lipidique ayant un point de fusion de 30 à 150°C et
d) 5 à 15% en poids d'un émulsifiant non ionique ayant une valeur HLB de 8 à 18
dans une matière véhicule liquide, comprenant
e) 0,1 à 35% en poids de silicones volatiles et
f) 5 à 25% en poids d'une huile hydrophile ou d'un mélange d'huiles hydrophiles.

2. Composition selon la revendication 1, **caractérisée en ce que** les polysaccharides sont choisis dans le groupe de l'amidon et de la cellulose et/ou de leurs dérivés.

3. Composition selon la revendication 1 ou 2, **caractérisée en ce que** l'amidon est modifié de façon hydrophobe.

4. Composition selon l'une quelconque des revendications 1 à 3, **caractérisée en ce que** la substance active anti-transpirante est un composé astringent d'aluminium et/ou d'aluminium-zirconium.

5. Composition selon l'une quelconque des revendications 1 à 4, **caractérisée en ce que** le composant lipidique ayant un point de fusion de 30 à 150°C est choisi dans le groupe formé par les triesters de glycérol avec les acides gras, les mono- et di-glycérides, les cires, les alcools gras et alcools de cire, les acides gras, les esters et/ou les éthers d'alcools gras et d'acides gras ainsi que les amides d'acides gras ou des mélanges quelconques de ces substances, tant que celles-ci fondent dans l'intervalle exigé de 30 à 150°C.

6. Composition selon la revendication 5, **caractérisée en ce que** le triglycéride d'acide gras ayant un point de fusion de 30 à 150°C est choisi parmi les triesters du glycérol avec les acides gras en C₁₈ à C₆₀, et en particulier les acides en C₁₈ à C₃₆, l'huile de ricin durcie, le tristéarate de glycérol, le tribéhénate de glycérol et le tripalmitate de glycérol.

7. Composition selon la revendication 5, **caractérisée en ce que** le composant lipidique ayant un point de fusion de 30 à 150°C est choisi parmi les alcools gras en C₁₄ à C₅₀ non ramifiés, en particulier les alcools gras en C₁₄ à C₃₀, de manière particulièrement préférée les alcools gras non ramifiés, saturés et non substitués, et les acides gras en C₁₂ à C₄₀ ou leurs mélanges, en particulier les acides laurique, tridécanoïque, myristique, pentadécanoïque, palmitique, margarique, stéarique, nonadécanoïque, arachique, béhénique, lignocérique, cérotique, mélissique, érucique et éléostéarique, ainsi que les acides gras substitués, l'acide 12-hydroxystéarique, et les amides ou monoéthanolamides des acides gras, ainsi qu'un mélange d'acides gras en C₂₀ à C₄₀, choisis de plus parmi les cires végétales naturelles, les cires animales, les cires minérales, les cires pétrochimiques, les cires chimiquement modifiées, et les cires synthétiques, les esters issus d'acides alcane-carboxyliques saturés et/ou insaturés, ramifiés et/ou non ramifiés, et d'alcools saturés et/ou insaturés, ramifiés et/ou non ramifiés, dans le groupe des esters issus d'acides carboxyliques, dicarboxyliques, tricarboxyliques aromatiques ou d'acides hydroxycarboxyliques (par exemple l'acide 12-hydroxystéarique) et d'alcools saturés et/ou insaturés, ramifiés et/ou non ramifiés, et de plus dans le groupe des lactides d'acides hydroxycarboxyliques à longue chaîne, en particulier les stéarates d'alkyle en C₁₆ à C₄₀, les stéarates d'alkyle en C₂₀ à C₄₀, les esters dialkyliques en C₂₀ à C₄₀ d'acides dimères, les hydroxystéaroylstéarates d'alkyle en C₁₈ à C₃₈, ou les érucates d'alkyle en C₂₀ à C₄₀, la cire d'abeille alkylique en C₃₀ à C₅₀, le citrate de tristéaryle, le citrate de triisostéaryle, l'heptanoate de stéaryle, l'octanoate de stéaryle, le citrate de trilauryle, le dipalmitate d'éthylèneglycol, le distéarate d'éthylèneglycol, le di(12-hydroxystéarate) d'éthylèneglycol, le stéarate de stéaryle, le stéarate de palmityle, le béhénate de stéaryle, l'ester cétylique, le béhénate de cétéaryle, le béhénate de béhényle, ainsi que parmi les cires de silicone.

8. Composition selon l'une quelconque des revendications 1 à 7, **caractérisée en ce que** le composant lipidique ayant un point de fusion de 30 à 150°C est contenu en une quantité totale de 0,1 à 40% en poids, de préférence de 1 à 20% en poids, et en particulier de 5 à 15% en poids.

9. Composition selon l'une quelconque des revendications 1 à 8, **caractérisée en ce que** la matière véhicule liquide est contenue en une quantité totale de 10 à 60% en poids, de préférence de 10 à 50% en poids, et en particulier de 20 à 45% en poids.

10. Composition selon l'une quelconque des revendications 1 à 9, **caractérisée en ce que** l'huile hydrophile est choisie parmi les alcanols, alcanediols, polyols, éthers-alcools, et éther-dialkyliques, ramifiés, liquides ayant chacun 12 à 40 atomes de C, les produits d'addition de 2 à 30 moles d'oxyde de propylène sur les alcanols mono- ou poly-valents ayant de 3 à 20 atomes de C, ainsi que les esters de l'acide carbonique, par exemple le carbonate de dioctyle, en outre parmi les produits d'addition liquides d'oxyde d'éthylène sur les alcanols ou sur les polyols, comme par exemple le glycérol, dans la mesure où ces produits ne tombent pas dans le groupe des émulsifiants ayant une valeur HLB de 8 à 18, encore parmi les alcools de Guerbet, qui présentent une ramification unique au niveau de l'atome de carbone β, par exemple le 2-hexyldécanol ou le 2-octyldodécanol, encore parmi l'isotridécanol, l'isohexadécanol et les mélanges de tels alcools.

11. Composition selon l'une quelconque des revendications 1 à 10, **caractérisée en ce que** l'émulsifiant non ionique ayant une valeur HLB de 8 à 18 est choisi parmi les produits d'addition de 10 à 50 moles d'oxyde d'éthylène, sur les alcools gras, les acides gras, les alcanolamides d'acides gras, les monoglycérides d'acides gras, les esters de sorbitane avec les acides gras, les esters de méthylglucoside avec les acides gras ou les polysiloxanes à modification polyglycoléther.

12. Composition selon l'une quelconque des revendications 1 à 11, **caractérisée en ce qu'**en outre au moins une charge inorganique y est contenue.

13. Composition selon l'une quelconque des revendications 1 à 12, **caractérisée en ce que** la charge inorganique est choisie dans le groupe des silicates.

14. Composition selon l'une quelconque des revendications 1 à 13, **caractérisée en ce qu'**elle présente une viscosité d'au moins 200 Pa.s à 25°C, mesurée avec un viscosimètre Brookfield-RVF, broche TE, Helipath, 4 tr/mn.

15. Utilisation d'une composition selon l'une quelconque des revendications 1 à 14, en tant qu'agent anti-perspirant pour application sur la peau.

16. Procédé pour préparer une composition selon l'une quelconque des revendications 1 à 14, **caractérisé en ce que** les composants de cire et d'huile sont fondus conjointement avec les émulsifiants sous agitation, ensuite les composants sous forme de poudre sont incorporés sous agitation, et on laisse refroidir la masse sous agitation constante à la température ambiante.
